# FASCICULE DE BREVET EUROPEEN

(11) **EP 1 455 596 B1**
(45) Date de publication et mention de la délivrance du brevet: **23.02.2011**
(21) Numéro de dépôt: 02795396.7
(22) Date de dépôt: 25.11.2002
(51) Int. Cl.: A23L 1/22, A23L 2/56, A23F 5/46, A23G 3/00

(54) **CAPSULE A SOLUBILISATION ET LIBERATION DU CONTENU RAPIDES**
KAPSEL MIT SCHNELLER LÖSLICHKEIT UND FREISETZUNG
CAPSULE WITH FAST CONTENT SOLUBILIZATION AND RELEASE

(30) Priorité: 26.11.2001 FR 0115277
(43) Date de publication de la demande: 15.09.2004
(73) Titulaire: V. Mane Fils, 06620 Le Bar-sur-Loup (FR)
(72) Inventeur: MANE, Jean, Domaine de Saint-Mathieu, F-06130 Grasse (FR); GRIMAULT, Pierre, F-06550 La Roquette sur Siagne (FR); HANNETEL, Jean-Michel, F-06130 Grasse (FR)
(74) Mandataire: Cabinet Plasseraud
(86) Numéro de dépôt international: PCT/FR2002/004034
(87) Numéro de publication internationale: WO 2003/045166

(56) Documents cités:
- US-A- 5 620 707
- US-B1- 6 238 690
- DATABASE WPI Section Ch, Week 198050 Derwent Publications Ltd., London, GB; Class B07, AN 1980-89057C XP002212315 & JP 55 138457 A (TOKAI CAPSEL KK), 29 octobre 1980 (1980-10-29)
- DATABASE WPI Section Ch, Week 197604 Derwent Publications Ltd., London, GB; Class A96, AN 1976-06281X XP002212316 & JP 50 106877 A (TOKAI CAPSULE KK), 22 août 1975 (1975-08-22)

## Description

La présente invention a pour objet une capsule à solubilisation et libération du contenu rapides.

Dans les domaines notamment des produits alimentaires et de l'« oral care » (c'est-à-dire des produits d'hygiène buccale), on cherche à administrer à un individu un composé tel qu'un principe actif ou un produit aromatique. Différents modes d'administration ont été développés, en fonction des applications : poudres, granules, capsules, microcapsules, liposomes, microsphères, etc.... Ces différents moyens présentent généralement tous des caractéristiques relatives à la protection et/ou à la libération du composé administré, adaptées au produit fini. Il peut s'agir notamment de résistance physique (mécanique, température...), chimique (pH, eau, force ionique...) ou enzymatique, ou de propriétés d'insolubilité ou de solubilité progressive (composés devant être avalés, composés gastro-résistants, ...) .

On s'intéressera plus particulièrement ici aux capsules ou microcapsules. Schématiquement, une capsule ou microcapsule comprend un coeur renfermant le ou les composés à véhiculer [le contenu], et une ou plusieurs enveloppes, souvent à base de gélatine. La différence entre une capsule et une microcapsule est la taille, plus ou moins importante. Dans la présente demande de brevet, on utilisera le terme capsule pour désigner les capsules et les microcapsules.

Les capsules connues dans l'art antérieur peuvent présenter certains problèmes de stabilité et d'étanchéité de l'enveloppe, avec notamment la migration vers l'extérieur de constituants du noyau ou la migration vers l'intérieur de constituants de l'enveloppe. D'autres problèmes rencontrés sont relatifs à l'incorporation d'un ou plusieurs des composants dans le coeur ou l'enveloppe de la capsule, ou encore le procédé de fabrication.

Les capsules rencontrées sont généralement avalées pour libérer un actif dans l'estomac par exemple, ou au contraire doivent être maintenues en bouche un certain temps afin de permettre à l'actif ou à l'arôme de se libérer.

On citera le brevet US 5,300,305 qui décrit une microcapsule pour hygiène buccale comprenant un noyau, entouré d'une enveloppe qui contient les ingrédients actifs difficilement solubles dans le noyau. Le brevet US 5,370,864 décrit une capsule pour hygiène buccale comprenant dans le noyau un composant particulier. Le brevet US 5,286,496 décrit une microcapsule pour hygiène buccale dont le noyau et l'enveloppe comprennent des actifs.

Certains brevets font référence à une capsule en plusieurs couches. Le document EP 1 020 177 décrit une capsule molle comprenant une première capsule dans une deuxième capsule, la deuxième capsule se solubilisant dans l'estomac. Le brevet US 6,200,603 décrit une capsule comprenant un noyau et une enveloppe pourvue d'un revêtement supplémentaire, qui stabilise la capsule et empêche la migration de certains composants entre les couches.

Le problème de la vitesse de l'éclatement d'une capsule a été étudié. En effet, pour certaines applications, il est préférable que l'éclatement de la capsule se fasse rapidement, afin que le ou les composés véhiculés soient disponibles rapidement.

On citera en particulier le brevet US 5,620,707 qui décrit de petites capsules dures appropriées pour aromatiser un breuvage, présentées comme se dissolvant rapidement dans le breuvage. Des capsules fabriquées selon ce brevet sont mentionnées ci-après, à titre d'exemple comparatif.

Cependant, en plus de la vitesse d'éclatement, il convient de prendre en compte la solubilisation de la capsule et plus particulièrement de l'enveloppe de la capsule. La question qui se pose est donc le devenir de l'enveloppe une fois qu'elle a éclaté et libéré son contenu. En effet, dans de nombreux cas l'enveloppe de la capsule est lentement ou imparfaitement insolubilisé en raison de la réticulation interne ou externe de la gélatine qu'elle comprend. Cet effet empêche ou ralentit la libération rapide du contenu de la capsule, et peut de surcroît provoquer une perception désagréable en bouche due aux résidus d'enveloppe non solubilisés, ce qu'on appellera ici l'« effet de peau ».

Or il est important, pour certaines applications, de s'affranchir de cet effet de peau, qui provoque une désagréable sensation en bouche.

Le brevet US 6,238,690 mentionne ce problème . Dans la description de l'art antérieur de ce brevet, il est précisé que les capsules sont classiquement formées en utilisant pour l'enveloppe des matériaux filmogènes du type gélatine, qui présentent l'inconvénient, outre la nécessité de recourir à un procédé de fabrication complexe, de se dissoudre lentement en laissant un résidu désagréable dans la bouche. Dans ce brevet, la solution apportée est d'utiliser comme matériau de l'enveloppe non pas un matériau filmogène mais un hydrate de carbone à l'état vitreux.

Cependant, le problème subsiste pour les capsules dont l'enveloppe comprend un matériau filmogène.

Après de longues recherches, la Demanderesse a conçu et mis au point une nouvelle capsule dont l'enveloppe comprend un polymère filmogène, à solubilisation et libération du contenu rapides. La capsule selon l'invention est solubilisée très rapidement et de surcroît ne présente aucun effet de peau ; la solubilisation de l'enveloppe est donc optimale. L'invention a donc pour objet une capsule comprenant un noyau et au moins une enveloppe comprenant au moins un polymère filmogéne, un agent plastifiant et un acide conformément à la revendication 1 caractérisée en ce qu'elle présente un temps de solubilisation totale de son enveloppe inférieur ou égal à 85 s selon un test A qui est défini ci-après.

On utilise dans ce test A un appareil de dissolution disposant d'une cellule à flux continu telle que décrite dans la pharmacopée française ou américaine (USP XXIII, 724). Cet appareil permet de modéliser la solubilité de la capsule dans la cavité buccale. On utilise préférentiellement une cellule à flux continu plutôt qu'un appareil classique de dissolution pour capsule en raison du fait que le contenu de la capsule peut être totalement lipophile et donc surnage parfois en surface et ne permet pas toujours d'obtenir des prélèvements homogènes au cours du temps.

Le mode opératoire utilisé dans le test A est le suivant : une capsule unique est introduite dans la cellule d'un appareil de dissolution tel que mentionné au paragraphe précédent, cellule qui est traversée par un flux aqueux maintenu à 37 +/- 0,5°C, pH 6,5, 50 ml/min. La capsule est alors observée à travers la cellule à l' oeil nu ou à l'aide d'une caméra associée à un logiciel d'analyse d'image (Microvision), permettant de définir un temps de percement (ouverture de l'enveloppe), un temps d'éclatement (libération totale de son contenu) et un temps de solubilisation totale (disparition de l'enveloppe de la capsule). Dans la présente demande de brevet, on entend par solubilisation la solubilisation de l'enveloppe de la capsule, encore appelé délitement au sens de la Pharmacopée.

Le temps de solubilisation totale de l'enveloppe d'une capsule selon l'invention est inférieur ou égal à 85 s selon le test A, de préférence inférieur ou égal à 80 s, de préférence encore inférieur ou égal à 70 s. Le temps d'éclatement d'une capsule selon l'invention est inférieur ou égal à 30 s, de préférence inférieur ou égal à 20 secondes, de préférence encore inférieur ou égal à 15 s.

Une telle capsule, lorsqu'elle est ingérée, génère donc une perception en bouche immédiate des composés actifs qu'elle contient.

Une capsule selon l'invention peut être utilisée en l'état, comme un produit fini ou une nouvelle forme galénique, ou encore comme ingrédient d'une autre forme galénique ou d'un autre produit. Elle peut êtres solubilisée après ingestion par l'utilisateur, ou solubilisée dans un milieu qui sera ingéré par l'utilisateur.

Une capsule selon l'invention n'est pas une capsule molle au sens de la technique ; il s'agit d'une capsule dure au toucher , qui peut se casser lorsqu'on la presse trop fortement entre les doigts. Sa dureté est de l'ordre de 1 à 5 kg/cm².

Dans un mode de réalisation, une capsule selon l'invention est sphérique ou sensiblement sphérique, de préférence parfaitement sphérique. Elle présente un diamètre variable, de préférence de 1 à 7 mm de diamètre. Ce diamètre dépendra de l'utilisation, et pourra être aisément choisi par l'homme de l'art. Le poids d'une capsule selon l'invention est variable, il peut être de 0,5 à 170 mg. Dans un mode de réalisation préféré, la capsule selon l'invention présente un diamètre de 4,5 à 6,5 mm et un poids de 45 à 140 mg ; par exemple un diamètre de 4,5 à 5,5 mm et un poids de 45 à 80 mg.

Une capsule selon l'invention est formée d'au moins une enveloppe fine et d'un noyau liquide. Elle peut être d'aspect brillant, transparente ou non. Le noyau et l'enveloppe peuvent être colorés, de la même couleur ou de couleurs différentes.

L'enveloppe de la capsule selon l'invention comprend au moins un polymère filmogène, choisi parmi les polymères filmogènes utilisés dans le domaine alimentaire ou pharmaceutique et connus de l'homme de l'art, en particulier les gélatines, les alcools polyvinyliques (PVA), les gommes naturelles (gomme arabique, guar, caroube, gellane, pullulane...), les carraghénanes, les dérivés cellulosiques, les dérivés de l'amidon, ....

Elle comprend de plus au moins un agent plastifiant, qui peut être de type glycérol, sorbitol, maltitol, triacétine, PEG, ou un autre polyol aux propriétés plastifiantes, et au moins un acide de type monoacide, diacide ou triacide , notamment les acides citrique, fumarique, malique, .... L'utilisation d'au moins un tel acide permet notamment d'assurer une stabilité microbiologique de l'enveloppe de la capsule et d'adapter ses propriétés physico-chimiques et sensorielles lors de sa dissolution (pH, solubilité, ...).

L'épaisseur de l'enveloppe de la capsule selon l'invention est comprise entre environ 30 et environ 100 µm, de préférence de 50 à 65 µm. L'enveloppe représente de 8 à 30% du poids de la capsule, de préférence 8 à 15%.

Le noyau de la capsule selon l'invention est préférentiellement composé de mélange de molécules hydrophobes ou partiellement solubles dans l'éthanol ou de molécules mises sous la forme d'émulsion huile/eau/huile. Il peut être composé d'un ou plusieurs solvants lipophiles classiquement utilisés dans les industries alimentaires, pharmaceutiques ou cosmétiques. Il s'agit en particulier des triglycérides, et notamment de triglycérides d'acide caprylique et caprique, des mélanges de triglycérides de type huile végétale, huile d'olive, de tournesol, de mais, d'arachide, de pépin de raisin, de germe de blé, des huiles minérales et des huiles de silicone. La quantité de solvant lipophile dans le noyau d'une capsule selon l'invention est de l'ordre de 0.01 à 90% du poids de la capsule, préférentiellement de 25 à 75%.

Le noyau peut également comprendre une ou des molécules aromatiques ou parfumantes telles que classiquement utilisées dans la formulation de compositions aromatisantes ou parfumantes. On citera notamment les hydrocarbures aromatiques, terpéniques et/ou sesquiterpéniques et plus particulièrement les huiles essentielles, les alcools, les aldéhydes, les phénols, les acides carboxyliques sous leur différentes formes, les éthers et acétals aromatiques, les hétérocycles azotés, les cétones, les sulfures, disulfures et mercaptans aromatiques ou non aromatiques. Il peut également comprendre une ou des molécules ou extraits à usage cosmétique.

Le noyau peut également comprendre un ou des agents dits alourdisseurs tels qu'utilisés dans les émulsions aromatiques. Nous citerons la gomme damar, les résines de bois type estergum, l'acétoisobutyrate de saccharose (SAIB) ou les huiles végétales bromées. La fonction de ces alourdisseurs est d'ajuster la densité du noyau liquide.

Le noyau peut également comprendre un ou plusieurs édulcorants, qui peuvent être apportés sous la forme d'une solution ou suspension dans l'éthanol. On citera à titre non exclusif l'aspartame, la NHDC, le sucralose, l'acésulfame, le néotame,...

Le noyau peut également comprendre un ou des agents aromatiques dit « sensate », qui apportent soit un effet rafraîchissant soit un effet chaud en bouche. On citera notamment comme agent rafraîchissant le succinate de menthyle et ses dérivés, notamment le Physcool^{®} commercialisé par la Société Demanderesse. On citera comme agent à effet chaud le vanillyl ethyl ether.

La composition exacte du noyau dépendra bien entendu de l'utilisation envisagée pour la capsule : alimentaire, hygiène buccale, pharmaceutique, cosmétique et donc du ou des composés actifs que l'on souhaite administrer à un individu, et pourra être aisément déterminée par l'homme de l'art.

Le noyau d'une capsule selon l'invention représente en poids 70 à 92% de la capsule, de préférence 80 à 92%, de préférence encore 85 à 92 %.

On connaît plusieurs procédés de fabrication de capsules. On citera les techniques de coacervation ou de polymérisation interfaciales, permettant d'obtenir des capsules généralement de petites tailles, généralement inférieures à 1 mm pour une forme parfaitement sphérique, les capsules de plus grosse granulométrie présentant des irrégularités de sphéricité. On peut citer également le procédé de fabrication de capsules molles permettant d'obtenir des capsules de plusieurs millimètres de diamètre obtenues par soudure de deux enveloppes hémisphériques.

Les capsules selon l'invention peuvent être préparées par co-extrusion. Le procédé de co-extrusion consiste à co-extruder deux liquides, l'un externe hydrophile (qui deviendra l'enveloppe), l'autre central lipophile (qui deviendra le noyau), dans un milieu organique huileux, ce qui conduit à la formation de capsules sphériques et sans joint de soudure.

Classiquement, les capsules, après co-extrusion, sont maintenues au froid pour assurer une bonne gélification de l'enveloppe, puis sont centrifugées pour éliminer le surplus d'huile, puis séchées et lavées à l'aide de solvant organique (acétone, acétate d'éthyle, éther de pétrole, ...) également pour éliminer le surplus d'huile. Dans le cas des capsules molles, les capsules sont traitées par immersion dans un liquide organique ou une émulsion contenant un agent de réticulation du type aldéhyde (formaldéhyde, glutaraldéhyde, ...) qui permet d'obtenir une dureté adéquate des capsules, puis séchées par un flux d'air à 25% d'humidité (voir le brevet US 2,578,943). Un tel procédé avec séchage utilisant de l'air sec ou du vide conduit néanmoins à une insolubilisation de l'enveloppe de gélatine par réaction de réticulation.

Les capsules selon l'invention peuvent être obtenues par le procédé suivant :
- co-extrusion des composants de l'enveloppe et des composants du noyau,
- éventuellement centrifugation,
- éventuellement une immersion des capsules obtenues dans un bain d'éthanol ou de solvant organique anhydre,
- séchage.

Plus précisément, après formation par co-extrusion, et après éventuellement centrifugation, les capsules sont éventuellement immergées dans un bain d'éthanol ou de tout autre solvant organique anhydre comme l'acétate d'éthyle ou l'isopropanol, maintenu à une température entre 0 et 25°C, plus particulièrement entre 10 et 20°C, permettant de laver les capsules de l'huile restant en surface puis de déshydrater de façon progressive, selon un équilibre osmotique, le film de gélatine. Ainsi, on évite les problèmes de réticulation et d'insolubilisation de l'enveloppe, donc l'effet de peau.

En sortant du bain de lavage, les capsules sont séchées dans un courant d'air aux caractéristiques d'humidité et de température contrôlée. L'humidité relative de l'air de séchage est compris entre 20 et 60%, préférentiellement entre 30 et 50%. La température de l'air de séchage est comprise entre 15 et 60°C, préférentiellement entre 35 et 45°C.

De plus, ce procédé permet d'obtenir des capsules parfaitement sphériques, et de taille très homogène.

### Exemple 1 :Capsule pour application buccale type « breathfreshener »

Une capsule de 4 mm de diamètre et de 38 mg, dont la composition est donnée dans le tableau 1, est analysée selon le test A. Différentes capsules obtenues sur le marché (capsule molle ou autre), de taille similaire, et pour une application identique, sont également testées selon le test A, à titre de comparatifs.

Les résultats sont donnés dans le tableau 2. La comparaison des temps de solubilisation totale et des temps d'éclatement (en secondes) selon le test A met en évidence la rapidité de solubilisation de la capsule selon l'invention.

Ces résultats sont confirmés par un panel de dégustateurs, qui observent les propriétés de solubilisation plus rapide et sans résidu de la capsule selon l'invention.

**Tableau 1**

| | |
|---|---|
| Gélatine | 7,7 % |
| Sorbitol | 1,2 % |
| Acide fumarique | 0,18 % |
| Blue FD&C #1 (commercialisé par WARNER JENKINSON) | 0,0001 % |
| Miglyol 812S (commercialisé par HULS) | 62,92 % |
| Arôme | 23,6 % |
| Physcool^{®} (agent rafraîchissant commercialisé par la Demanderesse) | 4,4 % |

**Tableau 2**

| | Temps d'éclatement de l'enveloppe | Temps de délitement ou de solubilisation de l'enveloppe |
|---|---|---|
| Capsule selon l'invention | 12 s | 66 s |
| Capsule 1 - capsule de co-extrusion sphérique de 5mm (Société Freund Ltd) | 86 s | 248 s |
| Capsule 2 - capsule de co-extrusion sphérique de 3,2 mm (Société Jintan, sous la marque Crystal Dew) | 34 s | 87 s |
| Capsule 3 - capsule molle type ovoïde 9 x 6 mm (Breathcaps de Breath Asure Inc.) | 75 s | 302 s |
| Capsule 4 - capsule molle type ovoïde 7.5 x 5.5 mm (Japan- Citrus) | 105 s | 333 s |

### Exemple 2 : Capsule arôme cannelle

Deux types de capsules selon l'invention, de 5 mm de diamètre, dont les compositions 1 et 2 sont données dans le tableau 3, sont analysées selon le test A. A titre d'exemple comparatif, une capsule de 5 mm, fabriquée selon l'exemple 2 du brevet US 5,620,707, est également testée selon le test A.

**Tableau 3**

| | Composition 1 (%) | Composition 2 (%) | Capsule selon US 5,620,707 (%) |
|---|---|---|---|
| Gélatine | 8,804 | 7,528 | 11,484 |
| Sorbitol (sol. 70%) | 0,978 | 0,836 | 4,362 |
| Acide fumarique | 0,196 | 0,167 | |
| Saccharine | | | 0,500 |
| Acésulfame | | | 0,644 |
| Aspartyl phényl alanine méthyl ester | | | 0,345 |
| Glycyrrhizin | | | 0,030 |
| Propylène glycol | | | 2,435 |
| Polyéthylène 400 | | | 25,820 |
| Rouge Allura (red 40) | 0,022 | 0,019 | 0,003 |
| Eau | 0,500 | 0,450 | 1,672 |
| Captex 300 (liponate) | 61,280 | 72,145 | 10,206 |
| Arôme cannelle | 22,500 | 11,029 | 15,250 |
| Isopropanol | | 7,280 | |
| Ethanol | 5,000 | | |
| Sucralose | 0,720 | 0,546 | |
| Sucrose acétate isobutyrate (SAIB) | | | 27,249 |

Les résultats des analyses de ces capsules selon le test A, ainsi que lors de l'analyse sensorielle, sont les suivants :

**Tableau 4**

| | Composition 1 | Composition 2 | Capsule selon US 5,620,707 |
|---|---|---|---|
| Temps d'éclatement | 12 +/- 3 s | 17 +/- 2 s | 48 +/- 3 s |
| Temps de solubilisation totale | 65 +/- 4 s | 70 +/- 2 s | 108 +/- 9 s |
| Temps de solubilisation totale après mise en bouche par évaluation sensorielle | 40 s | 35 s | > 2 min |

### Exemple 3 : Capsule à libération contrôlée de molécules aromatiques

Une capsule de 5 mm dont la composition est donnée dans le tableau 5 est analysée selon le test A.

**Tableau 5**

| | % | mg |
|---|---|---|
| Gélatine | 6.63 | 4.105 |
| Sorbitol | 1.78 | 1.103 |
| Acide fumarique | 0,14 | 0.088 |
| Miglyol 812S | 72.98 | 45.21 |
| Thymol . | 2.58 | 1.60 |
| Menthol | 6.28 | 3.89 |
| Arôme commercialisé par la Demanderesse | 7.35 | 4.56 |
| Physcool^{®} | 2.26 | 1.40 |
| TOTAL | 100 | 61.95 |

Le prélèvement de différents échantillons permet d'établir la cinétique in vitro de libération de molécules aromatiques dans l'eau à 37°C +/- 1°C et à pH 6,5. Le tableau 6 indique la concentration en molécules aromatiques des différents échantillons. On met ainsi en évidence une rapidité de libération de l'ordre de quelques secondes.

**Tableau 6**

| Temps (secondes) | Thymol | Menthol | Arôme MANE |
|---|---|---|---|
| 0-5 | 19 | 47 | 32 |
| 5-10 | 28 | 67 | 46 |
| 10-15 | 22 | 63 | 50 |
| 15-20 | 31 | 90 | 69 |
| 20-30 | 20 | 62 | 57 |
| 30-40 | 7 | 24 | 21 |
| 40-50 | 7 | 24 | 23 |
| 50-60 | 4 | 16 | 16 |
| 60-70 | 1 | 6 | 5 |
| Total | 139 ppm | 399 ppm | 319 ppm |

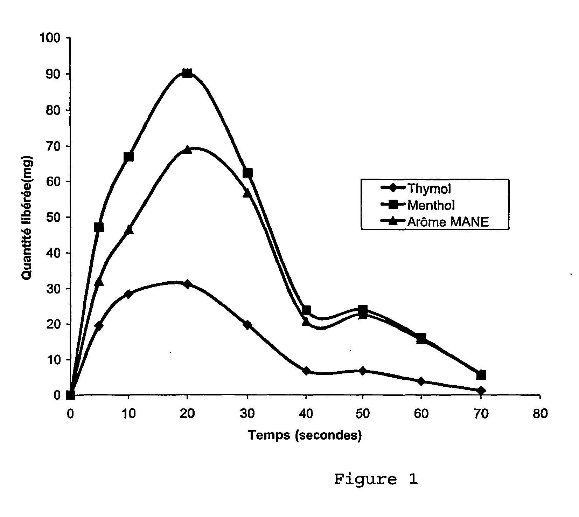

Les courbes de libération de chaque molécule aromatique (en mg) en fonction du temps (en seconde) sont données en Figure 1. On peut constater que la plus grande partie des molécules est libérée dans les 40 s, au plus dans les 70 s.

### Exemple 4 : capsule comme noyau d'un produit de confiserie

Une capsule de 4 mm et de 38 mg dont la composition est donnée dans le tableau 7 est utilisée comme noyau pourvu d'un enrobage de dragéification pour obtenir un produit de confiserie pour hygiène buccale.

**Tableau 7**

| | |
|---|---|
| Gélatine | 10 % |
| Sorbitol | 1,2 % |
| Acide fumarique | 0,2% |
| Blue FD&C #1 | 0,001% |
| Miglyol 812S | 66,099 % |
| Arôme et actifs | 20 % |
| Physcool ® | 2 % |
| Vitamine E | 0,5 % |

La capsule selon l'invention est rapidement solubilisée en bouche après dissolution de l'enveloppe enrobante . L'enveloppe enrobante est obtenue par dragéification dans une turbine à dragéifier de la capsule en utilisant les matières premières suivantes : maltitol, gomme arabique, gomme shellac, huile végétale, dioxyde de titane, Arôme poudre MSD Menthol commercialisé par la demanderesse. Il est observé que la capsule ne laisse pas d'effet de peau en fin de dégustation et laisse un fort impact aromatique en bouche.

### Exemple 5 : capsule en application boisson

Une capsule de 1 mm et de 0,53 mg selon l'invention dont la composition est donnée dans le tableau 8 est incorporée à un café instantané en poudre.

**Tableau 8**

| | |
|---|---|
| Gélatine | 15 % |
| Gomme arabique | 9,89 % |
| Sorbitol | 3 % |
| Acide fumarique | 0,01 % |
| Caramel colorant | 0,1 % |
| Huile végétale | 36 % |
| Arôme café volatile commercialisé par la Société Demanderesse | 36 % |

On verse 100 ml d'eau chaude à 65°C. Au bout de 10 secondes, la capsule se solubilise et libère une forte odeur de café agréable. L'enveloppe de la capsule est totalement solubilisée au bout de 30 secondes.

### Exemple 6 : capsule en application salée

Une capsule de 4 mm et de 38mg selon l'invention dont la composition est donnée dans le tableau 9 est incorporée à 0,5% à un mélange en poudre pour soupe instantanée.

**Tableau 9**

| | |
|---|---|
| Gélatine | 7 % |
| Gomme arabique | 4 % |
| Sorbitol | 1.5 % |
| Acide citrique | 0,5 % |
| Huile de tournesol | 60 % |
| Arôme poulet commercialisé par la Société Demanderesse | 27 % |

On verse 200 ml d'eau chaude à 65°C. Au bout de 10 secondes, la capsule se solubilise et libère une forte odeur de poulet agréable. L'enveloppe de la capsule est totalement solubilisée au bout de 30 secondes.

## Revendications

1. Capsule sphérique et sans joint de soudure comprenant un noyau et au moins une enveloppe comprenant au moins un polymère filmogène choisi dans le groupe consistant en les gélatines, les alcools polyvinyliques (PVA), les gommes naturelles, les carraghénanes, les dérivés cellulosiques, les dérivés de l'amidon, un agent plastifiant choisi parmi le glycérol, le sorbitol, le maltitol, la triacétine et le PEG , et un acide de type monoacide, diacid ou triacide, ladite capsule présentant un temps de solubilisation totale de son enveloppe inférieur ou égal à 85 s selon un test

2. Capsule selon la revendication 1, **caractérisée en ce que** le temps de solubilisation totale de son enveloppe est inférieur ou égal à 80 s, de préférence inférieur ou égal à 70 s.

3. Capsule selon l'une ou l'autre des revendications 1 et 2, **caractérisée en ce qu'**elle est sphérique ou sensiblement sphérique, et présente un diamètre de 1 mm à 7 mm.

4. Capsule selon l'une quelconque des revendications précédentes, **caractérisée en ce que** le noyau comprend un ou plusieurs solvants lipophiles, éventuellement une ou des molécules aromatiques ou parfumantes, éventuellement une ou des molécules ou extraits à usage cosmétique, éventuellement un ou des agents dits alourdisseurs, éventuellement un ou plusieurs édulcorants, éventuellement un ou des agents dit sensate rafraîchissant ou à effet chaud.

5. Capsule selon l'une quelconque des revendications précédentes, **caractérisée en ce que** le noyau représente en poids 70 à92% de la capsule, de préférence 80 à 92%, de préférence encore 85 à 92%.

6. Procédé de fabrication de la capsule selon l'une quelconque des revendications 1 à 5 , **caractérisé en ce qu'**il comprend les étapes suivantes :
- co-extrusion des composants de l'enveloppe et des composants du noyau,
- éventuellement centrifugation,
- éventuellement une immersion des capsules obtenues dans un bain d'éthanol ou de solvant organique anhydre,
- séchage.

7. Produit comprenant une capsule selon l'une quelconque des revendications 1 à 5.

8. Produit selon la revendication 7 , **caractérisé en ce qu'**il s'agit d'un produit d'hygiène buccale, d'un produit alimentaire, d'un produit pharmaceutique ou d'un produit à usage cosmétique.

## Claims

1. Spherical capsule with no welding joint comprising a core and at least one envelope comprising at least one film-forming polymer chosen in the group consisting of gelatins, polyvinyl alcohols, (PVAS), natural gums, carreghenans, cellulose derivatives, starch derivatives, a plasticizer chosen among glycerol, sorbitol, maltitol, triacetine and PEG, and an acid of the monoacid, diacid or triacid type, said capsule exhibiting a total solubilization time of its envelope of less than or equal to 85 s, according to a test A.

2. Capsule according to claim 1, **characterized in that** the total solubilization time of its envelope is less than or equal to 80 s, preferably less than or equal to 70 s.

3. Capsule according to either of claims 1 and 2, **characterized in that** it is spherical or substantially spherical, and is 1 mm to 7 mm in diameter.

4. Capsule according to any one of the preceding claims, **characterized in that** the core comprises one or more lipophilic solvents, optionally one or more aromatic or fragrancing molecules, optionally one or more molecules or extracts for cosmetic use, optionally one or more "weighting" agents, optionally one or more sweeteners, optionally one or more "sensate" freshening agents or agents with a hot effect.

5. Capsule according to any one of the preceding claims, **characterized in that** the core represents by weight 70 to 92% of the capsule, preferably 80 to 92%, more preferably 85 to 92%.

6. Method of producing the capsule according to any one of claims 1 to 5, **characterized in that** it comprises the following steps:
- co-extrusion of the components of the envelope and of the components of the core,
- optionally centrifugation,
- optionally immersion of the capsules obtained in a bath of ethanol or of anhydrous organic solvent,
- drying.

7. Product comprising a capsule according to any one of claims 1 to 5.

8. Product according to Claim 7, **characterized in that** it is an oral hygiene product, a food product, a pharmaceutical product or a product for cosmetic use.

## Patentansprüche

1. Sphärische Kapsel ohne Schweißnaht, umfassend einen Kern und mindestens eine Hülle, welche mindestens ein filmbildendes Polymer, ausgewählt aus der Gruppe, bestehend aus Gelatinen, Polyvinylalkoholen (PVA), natürlichen Gummen, Carragenen, Cellulosederivaten, Stärkederivaten, einem Weichmacher, ausgewählt aus Glycerin, Sorbit, Maltit, Triacetin und PEG, und einer Säure vom Monosäure-, Disäure- oder Trisäure-Typ, umfasst, wobei die Kapsel eine Gesamtsolubisilierungszeit ihrer Hülle von kleiner als oder gleich 85 s gemäß einem Test A aufweist.

2. Kapsel gemäß Anspruch 1, **dadurch gekennzeichnet, dass** die Gesamtsolubisilierungszeit ihrer Hülle kleiner als oder gleich 80 s, bevorzugt kleiner als oder gleich 70 s ist.

3. Kapsel gemäß einem der Ansprüche 1 oder 2, **dadurch gekennzeichnet, dass** sie sphärisch oder im Wesentlichen sphärisch ist und einen Durchmesser von 1 mm bis 7 mm aufweist.

4. Kapsel gemäß einem der vorhergehenden Ansprüche, **dadurch gekennzeichnet, dass** der Kern ein oder mehrere lipophile Lösungsmittel, gegebenenfalls ein oder mehrere aromatische oder parfümierende Moleküle, gegebenenfalls ein oder mehrere Moleküle oder Extrakte zur kosmetischen Verwendung, gegebenenfalls ein oder mehrere sogenannte Beschwerungsmittel, gegebenenfalls ein oder mehrere Süßungsmittel, gegebenenfalls ein oder mehrere Mittel, die eine kühlende Empfindung hervorrufen oder mit einem heißen Effekt, umfasst.

5. Kapsel gemäß einem der vorhergehenden Ansprüche, **dadurch gekennzeichnet, dass** der Kern 70 bis 92% an Gewicht der Kapsel, bevorzugt 80 bis 92%, stärker bevorzugt 85 bis 92% aufweist.

6. Verfahren zur Herstellung der Kapsel gemäß einem der Ansprüche 1 bis 5, **dadurch gekennzeichnet, dass** es die folgenden Schritte umfasst:
- Coextrusion der Bestandteile der Hülle und der Bestandteile des Kerns,
- gegebenenfalls Zentrifugation,
- gegebenenfalls Eintauchen der erhaltenen Kapseln in einem Bad aus Ethanol oder wasserfreiem organischen Lösungsmittel,
- Trocknen.

7. Produkt, umfassend eine Kapsel gemäß einem der Ansprüche 1 bis 5.

8. Produkt gemäß Anspruch 7, **dadurch gekennzeichnet, dass** es sich um ein Produkt der Mundhygiene, ein Nahrungsmittelprodukt, ein pharmazeutisches Produkt oder ein Produkt zur kosmetischen Verwendung handelt.
